# EUROPEAN PATENT APPLICATION

(11) **EP 3 037 520 A1**
(43) Date of publication of application: **29.06.2016**
(21) Application number: 15201475.9
(22) Date of filing: 21.12.2015
(51) Int. Cl.: C12N 1/20, C12P 7/06, C12P 7/54

(54) **FERMENTATIONS OF ACETOGENIC BACTERIA WITH SPECIFIC CYSTEINE CONCENTRATIONS**

(30) Priority: 22.12.2014 EP 14199536
(71) Applicant: Evonik Degussa GmbH, 45128 Essen (DE)
(72) Inventor: Haas, Thomas, 48161 Münster (DE); Bülter, Thomas, 47279 Duisburg (DE); Demler, Martin, 46286 Dorsten (DE); Thiessenhusen, Anja, 48147 Münster (DE)

(57) **Abstract**

The present invention relates to a method of culturing at least one acetogenic bacteria, the method comprising growing the bacteria in an aqueous medium comprising an amount of cysteine and/or cystine, wherein the cysteine and/or cystine concentration in the medium is maintained at a concentration less than 0.20 g/L.

## Description

### FIELD OF THE INVENTION

The present invention relates to a method of culturing acetogenic bacteria. In particular, the method comprises growing the bacteria in suitable conditions for production of ethanol and/or acetate from a carbon source.

### BACKGROUND OF THE INVENTION

Biotechnological production of alcohols is of great interest and use in the current world, especially in the production of biofuels. These alcohols may also be used in downstream processes to produce organic compounds.

Numerous conventional methods exist for sustaining microorganism culture that is capable of producing alcohols. However, these methods suffer from numerous inefficiencies. Several of these microorganisms that produce alcohol for example acetogenic bacteria are delicate by nature and susceptible to slight changes in the surrounding conditions in the cultural medium. This reduces the efficiency of production of alcohols from a suitable carbon source. There thus remains a need for additional more effective methods for sustaining acetogenic microorganism cultures in an aqueous medium.

In particular, there remains a need in the art in preserving culture in syngas fermentation processes in suitable conditions so that the acetogenic bacteria will function at its optimised level. There is a need to sustain cultures in the event of various interruptions in industrial process of alcohol production.

### DESCRIPTON OF THE INVENTION

The present invention attempts to solve the problem above by providing a suitable method for culturing acetogenic microorganism that allows the bacteria to produce alcohol and/or acetate efficiently. In particular, the present invention provides at least one method of culturing acetogenic bacteria in a medium that comprises an amount of cysteine and/or cystine. The cysteine and/or cystine may be present in the medium at a low concentration and the concentration may be maintained throughout the production process or the fermentation process.

According to one aspect of the present invention, there is provided a method of culturing at least one acetogenic microorganism, the method comprising growing the bacteria in an aqueous medium comprising an amount of cysteine and/or cystine wherein the concentration of cysteine and/or cystine is maintained at a concentration about less than or equal to 0.20 g/L.

According to any aspect of the present invention, there is provided a method of producing acetate and/or ethanol from at least one carbon source, the method comprising the step of growing at least one acetogenic bacteria in an aqueous medium comprising an amount of cysteine and/or cystine, wherein the concentration of cysteine and/or cystine in the medium is maintained at a constant concentration less than or equal to 0.20 g/L.

The term "acetogenic microorganism" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. The acetogenic microorganism may be any microorganism that may be autotrophically cultured. Examples include but are not limited to *Acetoanaerobium notera (ATCC 35199), Acetonema longum (DSM 6540), Acetobacterium carbinolicum (DSM 2925), Acetobacterium malicum (DSM 4132), Acetobacterium species no. 446 (*Morinaga et al., 1990, J. Biotechnol., Vol. 14, p. 187-194*),Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950, formerly Ruminococcus productus, formerly Peptostreptococcus productus), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539)*, *Clostridium methoxybenzovorans (DSM 12182), Clostridium ragsdalei (DSM 15248), Clostridium scatologenes (DSM 757), Clostridium species ATCC 29797 (*Schmidt et al., 1986, Chem. Eng. Commun., Vol. 45, p. 61-73*), Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1 (*Sakai et al., 2004, Biotechnol. Let., Vol. 29, p. 1607-1612*), Moorella thermoacetica (DSM 521, formerly Clostridium thermoaceticum), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440), Thermoanaerobacter kivui (DSM 2030, formerly Acetogenium kivui).*

The term 'culturing' as used herein refers to growing microorganisms under artificially controlled conditions. A culturing procedure may be conducted using a suitable medium and culturing conditions well known in the art. Those skilled in the art can readily control the culturing procedure to correspond to the strains employed. For example, it may be performed in a batch type, in a continuous type, or in a fed-batch type, but is not limited thereto. In particular, culturing refers to the growing of acetogenic microorganisms in a culture medium as well as to a process wherein the cells do not grow but assimilate catabolise or convert substrates provided in the culture medium. The term 'culture medium' is used interchangeably with the term 'aqueous medium'. The culture medium comprises essentially all nutrients and physical growth factors necessary for the growth, replication, maintenance, viability or activity of the microorganism being cultured. The term includes reference to a fresh culture medium in which no cell has grown, as well as to a "spent" culture medium from which the cells have been removed. In any case, the culture medium comprises cysteine and/or cystine.

The terms "culture" and "cell culture" as used herein refer to a cell population that is suspended in a cell culture medium under conditions suitable to survival and/or growth of the cell population. As used herein, these terms may refer to the combination comprising the cell population (e.g., the acetogenic microorganisms) and the medium in which the population is suspended.

The term 'cysteine and/or cystine' as used herein refers to the amino acid cysteine or a synthetic analogue thereof, wherein the analogue contains a free sulfhydryl group and/or the oxidised cysteine. In particular, cysteine is an α-amino acid with the chemical formula HO₂CCH(NH₂)CH₂SH. It is a semi-essential amino acid and the thiol side chain in cysteine often participates in enzymatic reactions, serving as a nucleophile. Cysteine has an amphoteric character. Cysteine and/or cystine may be included in the medium according to any aspect of the present invention using a compound selected from the group consisting of L-cysteine-HCl, L-Cysteine, L-Cysteine-HCl (free of water), L-Cysteine*H₂O and the like. The cysteine and/or cystine in the medium according to any aspect of the present invention may be about less than or equal to 0.20 g/L. The concentration of cysteine and/or cystine in the medium may thus be any value about below or equal to 0.20g/L but greater than 0.00g/L. In one example, the concentration of cysteine in the medium may be about 0.21g/L, 0.22g/L, 0.23g/L, 0.24g/L, 0.20g/L, 0.19g/L, 0.18g/L, 0.17g/L, 0.16g/L, 0.15g/L, 0.14g/L, 0.13g/L, 0.12g/L, 0.11g/L, 0.10g/L, 0.09g/L, 0.08g/L, 0.07g/L, 0.06g/L, 0.05g/L, 0.04g/L, 0.03g/L, 0.02g/L, 0.01g/L, or the like. In particular, the concentration of cysteine and/or cystine in the medium may be a range selected from any one of the concentrations listed here. For example, the concentration of cysteine and/or cystine in the medium according to any aspect of the present invention may be in the range of 0.01g/L to 0.24g/L, 0.01g/L to 0.20g/L, 0.05g/L to 0.20g/L, 0.10g/L to 0.20g/L, 0.15g/L to 0.20g/L, and the like. In one example, the concentration of cysteine and/or cystine in the medium according to any aspect of the present invention may be less than 0.40g/l, in particular less than 0.25g/L but greater than 0.00g/L. According to any aspect of the present invention, the aqueous medium comprises an amount of cysteine and/or cystine which refers to cysteine and/or cystine being present in the medium at a concentration at least above 0.00g/L. Accordingly, there is always cysteine and/or cystine present in the medium used according to any aspect of the present invention.

As used herein, the terms "about" and "approximately", as applied to one or more particular cell culture conditions refer to a range of values that are similar to the stated reference value for that culture condition or conditions. In certain examples, the term "about" refers to a range of values that fall within 25, 20, 19, 18, 17, 16, 15, 14, 13, 12, 11, 10, 9, 8, 7, 6, 5, 4, 3, 2, 1 percent or less of the stated reference value for that culture condition or conditions. For example, an amount of an ingredient employed in a mixture when modified by "about" includes the variation and degree of care typically employed in measuring in an experimental condition in production plant or lab. For example, the amount of a component of a product when modified by "about" includes the variation between batches in multiple experiments in the plant or lab and the variation inherent in the analytical method. Whether or not modified by "about," the amounts include equivalents to those amounts. Any quantity stated herein and modified by "about" can also be employed in the present invention as the amount not modified by "about." In particular, the term 'about 0.20g/L of cysteine and/or cystine' may refer to the concentration of 0.16g/L, 0.17g/L, 0.18g/L, 0.19g/L, 0.20g/L, 0.21g/L, 0.22g/L, 0.23g/L, 0.24g/L and the like. In another example, the term 'about 0.10g/L of cysteine and/or cystine' may refer to the concentration of 0.15g/L, 0.16g/L, 0.17g/L, 0.18g/L, 0.19g/L, 0.05g/L, 0.06g/L, 0.07g/L, 0.08g/L, 0.09 g/L and the like. In another example, the term 'about 0.08g/L of cysteine and/or cystine' may refer to the concentration of 0.081g/L, 0.082g/L, 0.083g/L, 0.084g/L, 0.075g/L, 0.076g/L, 0.077g/L, 0.078g/L, 0.079g/L and the like.

The concentration of cysteine and/or cystine in the medium used according to any aspect of the present invention may be maintained to maintain the ethanol productivity. "Ethanol productivity" is the volumetric productivity of ethanol, calculated as the ratio of the steady state ethanol concentration and the liquid retention time (LRT) in continuous systems, or the ratio of the ethanol concentration and the time required to produce that concentration in batch systems. The phrase "high ethanol productivity" describes a volumetric ethanol productivity of greater than 10 g/L/day. The concentration of the cysteine and/or cystine in the culture medium used according to any aspect of the present invention may be maintained at the specific concentrations mentioned. In one example, the concentration of cysteine and/or cystine is at least substantially maintained at the concentration mentioned above, for example at least above 0.0g/L and equal to or below about 0.20g/L, 0.10g/L, 0.08g/L and the like. A skilled person would be capable of maintaining the concentration of cysteine and/or cystine at the desired level by methods known in the art. In particular, the skilled person would regularly measure the concentration of cysteine and/or cystine in the culture medium and adjust the concentration of cysteine and/or cystine accordingly by adding a higher or lower concentration of cysteine and/or cystine into the medium. In one example, the cysteine and/or cystine may be added to the aqueous medium in in a continuous flow separately from the continuous feed of the aqueous medium. In another example, cysteine and/or cystine may be part of the culture medium that is being topped up. In particular, cysteine and/or cystine may be fed to the aqueous medium as part of the nutrient feed or separately. Whichever route is taken to feed cysteine and/or cystine to the aqueous medium, a skilled person would understand the means to maintain the concentration of cysteine and/or cystine in the aqueous medium. In one example, the cysteine and/or cystine concentration in the medium may be maintained by topping up the cysteine and/or cystine every about 20 hours of fermentation. In another example, the top up of cysteine and/or cystine in the medium may take place every about 5, 10, 15, 20, 25, 30 hours from the beginning of the culturing and/or fermentation process.

In one example, the concentration of cysteine and/or cystine in the aqueous medium is maintained at any of the concentrations used according to any aspect of the present invention for 80% of the reaction period. In another example, the concentration of cysteine and/or cystine is maintained for 50%, 55%, 60%, 65%, 70%, 75%, 85%, 90%, 95% or 100% of the reaction time. In this regard, 'reaction time' refers to the period during with a process takes place. In particular, reaction time refers to the period of time from when a reaction starts to when the reaction ends and/or is completed, i.e. where the substrate is used up. In one example, the substrate may be synthesis gas, and the reaction is completed when the synthesis gas in the fermenter is used up and the reaction stops and no further synthesis gas is fed into the fermenter. Therefore, the reaction time refers to the period from which the fermentation starts (i.e. when the syngas first comes into contact with at least one acetogenic bacteria in a fermenter in suitable fermentation conditions) to when the fermentation ends (i.e. when there is no more syngas in the fermenter and/or when there is another limiting factor in the fermenter that stops the reaction from continuing). In one example, the reaction period may be for 24hr, 42hr, 72hr, 96hr and the like. In another example, the reaction period may be for 90, 91, 92, 93, 94, 95, 97 and the like hours.

The term "acetate" as used herein, refers to both acetic acid and salts thereof, which results inevitably, because as known in the art, since the microorganisms work in an aqueous environment, and there is always a balance between salt and acid present.. The ratio of molecular acetic acid to acetate is dependent upon the pH of the system, i.e., at a constant "acetate" concentration, the lower the pH, the higher the molecular acetic acid concentration relative to acetate salt.

According to any aspect of the present invention, the aqueous medium may further comprise a carbon source comprising CO and/or CO₂.

The carbon source may be synthesis gas. Synthesis gas can for example be produced as a by-product of coal gasification. Accordingly, the microorganism of the mixed culture according to any aspect of the present invention may be capable of converting a substance which is a waste product into a valuable resource.

In another example, synthesis gas may be a by-product of gasification of widely available, low-cost agricultural raw materials for use with the mixed culture of the present invention to produce substituted and unsubstituted organic compounds.

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, H₂ and CO₂. In one example, the carbon source used according to any aspect of the present invention comprises at least 50% by weight of CO and/or CO₂. In another examples, the carbon source used according to any aspect of the present invention comprises at least 30% by weight of CO and/or CO₂

Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

Mixtures of sources can be used as a carbon source.

According to any aspect of the present invention, a reducing agent, for example hydrogen may be supplied together with the carbon source. In particular, this hydrogen may be supplied when the C and/or CO₂ is supplied and/or used. In one example, the hydrogen gas is part of the synthesis gas present according to any aspect of the present invention. In another example, where the hydrogen gas in the synthesis gas is insufficient for the method of the present invention, additional hydrogen gas may be supplied.

A skilled person would understand the other conditions necessary to carry out the method of the present invention. In particular, the conditions in the container (e.g. fermenter) may be varied depending on the first and second microorganisms used. The varying of the conditions to be suitable for the optimal functioning of the microorganisms is within the knowledge of a skilled person.

In one example, the method of the present invention may be carried out in an aqueous medium with a pH between 5 and 8, 5.5 and 7. The pressure may be between 1 and 10 bars.

The term "fermenter," as used herein may include a fermentation device consisting of one or more vessels and/or towers or piping arrangement, which includes the Continuous Stirred Tank Reactor (CSTR), Immobilized Cell Reactor (ICR), Trickle Bed Reactor (TBR), Bubble Column, Gas lift Fermenter, Static Mixer, or other device suitable for gas-liquid contact. In particular, the fermenter may comprise a growth reactor which feeds the fermentation broth to a second fermenter, in which most of the product, ethanol, is produced.

According to another aspect of the present invention, there is provided a method of fermenting syngas to produce acetate and/or ethanol, the method comprising culturing at least one acetogenic bacteria in an aqueous medium comprising cysteine and/or cystine at a concentration less than 0.20 g/L.

The term "fermentation" means fermentation of CO to alcohols and acetate. In particular, fermentation may refer to the fermentation of syngas to produce ethanol and/or acetate. This method can be carried out in a fermenter using a continuous method. The term "continuous method" as used herein refers to a fermentation method which includes continuous nutrient feed, substrate feed, cell production in the fermenter, cell removal (or purge) from the fermenter, and product removal. This continuous feeds, removals or cell production may occur in the same or in different streams. A continuous process results in the achievement of a steady state within the fermenter. By "steady state" is meant that all of these measurable variables (i.e., feed rates, substrate and nutrient concentrations maintained in the fermenter, cell concentration in the fermenter and cell removal from the fermenter, product removal from the fermenter, as well as conditional variables such as temperatures and pressures) are constant over time. In particular, the cysteine concentration may be maintained in the fermenter.

### BRIEF DESCRIPTION OF THE FIGURES

No figures.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### EXAMPLE 1

### Production autotrophs in carbonate buffer of C. ljungdahlii and synthesis gas with varying concentrations of L-Cysteine HCl

The wild-type strain *Clostridium ljungdahlii* is used under growth-limiting conditions for autotrophic product formation in carbonate buffer with different concentrations of L-cysteine hydrochloride.

For the production, anaerobic carbonate buffer (adjusted with KOH 1.4 g /L) was used. The only addition to the buffer was L-cysteine HCl at different concentrations.

For identical autotrophic productions, 500 mL septum bottles were filled with 100 mL carbonate buffer and shaken at 150 min⁻¹ in an open shaking water bath shaker Innova 3100 by New Brunswick Scientific. The reactions were carried out at 37 °C with manual control of the pH. Once the pH dropped below 5.5, KOH was replenished from a stock solution of 140 g/L. In the present experiments the pH in both reactors were checked at the following points in time 50.3 h, 73.3 h and corrected by the addition of each 1.4 g/L KOH manually.

The reactors were inoculated with a synthesis gas mixture of 67 % H₂ and 33 % CO₂ and pressure of 0.8 bar applied. To replenish the depleted gas, the gas above the reactors was replaced once a day and again the pressure brought to 0.8 bars.

The reactor 1 contained a concentration of L-cysteine- HCl 0.1 g/L, and reactor 2 contained a concentration of L-cysteine HCl of 0.4 g / L.

The reactor was started by seeding autotrophic grown cells at an OD of 0.2. The preculture was carried out continuously in a 1L bottle with 500 mL complex medium. The complex medium consisting of 1 g/l NH₄Cl, 0,1 g/l KCI, 0,2 g/l MgSO₄ x 7 H₂O, 0,8 g/l NaCl, 0,1 g/l KH₂PO₄, 20 mg/l CaCl₂ x 2 H₂O, 20 g/l MES, 1 g/l yeast extract, 0,4 g/L L-Cysteine-HCl, 20 mg/l nitrilotriacetic acid, 10 mg/l MnSO₄ x H₂O, 8 mg/l (NH₄)₂Fe(SO₄)₂ x 6 H₂O, 2 mg/l CoCl₂ x 6 H₂O, 2 mg/l ZnSO₄ x 7 H₂O, 0,2 mg/l CuCl₂ x 2 H₂O, 0,2 mg/l Na₂MoO₄ x 2 H₂O, 0,2 mg/l NiCl₂ x 6 H₂O, 0,2 mg/l Na₂SeO₄, 0,2 mg/l Na₂WO₄ x 2 H₂O, 20 µg/l d-Biotin, 20 µg/l folic acid, 100 µg/l pyridoxine HCl, 50 µg/l thiamine-HCl x H₂O, 50 µg / l of riboflavin, 50 µg / l nicotinic acid, 50 g / l Ca-pantothenate, 1 µg / l vitamin B12, 50 µg / l p-aminobenzoate, 50 µg / l lipoic acid. The culture was then gassed continuously with synthesis gas (67% H₂, 33% CO₂) at a flow rate of 3 l / h over a filter with pore size 10 microns. The cells were in late log phase growth at an OD of 0.37 and where anaerobically centrifuged (4500 rpm, 4300 g, 20 °C, 10 min). The supernatant was discarded and the cell pellet resuspended in 10 ml carbonate buffer. The prepared cells were then used to inoculate the actual tests.

When sampling each 5 ml sample was removed for determination of OD600, pH and the product spectrum. The determination of the product concentration was performed using quantitative 1H-NMR spectroscopy. As an internal quantification standard sodium trimethylsilylpropionate was used (T(M)SP).

The gas consumption in both reactors was measured from the start of gas consumption. The total gas consumption in reactor 1 with an L-cysteine-HCl concentration of 0.1 g / L over the test period of 92.3 h was higher than in the reactor with an L-cysteine-HCl-concentration of 0.4 g / L. The concentrations of ethanol and acetate in the mixture with an L-cysteine-HCl-concentration of 0.1 g / L was higher than in the mixture with L-cysteine-HCl-concentration of 0.4 g / L after 92.3 h (Table 1).

**Table 1. Results of acetate and ethanol production with different cysteine concentrations. (n.d. not detected)**

| | | | | | **NMR** | | |
|---|---|---|---|---|---|---|---|
| **Reactor** | **Time, h** | Δ**p, bar** | **pH** | **OD600** | **Ethanol, mg/L** | **Acetate, mg/L** | **Cysteine, mg/L** |
| **1** | 0,0 | - | 6,47 | 0,25 | 6 | 13 | 199 |
| MG-13-COx-062-P2 | 24,5 | 0,6 | 5,63 | 0,25 | 19 | 1418 | 92 |
| | 50,3 | 0,6 | 4,71 | 0,30 | 130 | 2556 | n.d. |
| KOH with 0,1 g/L L-Cysteine-HCl | 73,3 | 0,6 | 4,83 | 0,30 | - | - | |
| | 92,3 | 0,4 | 5,90 | 0,26 | 321 | 6960 | n.d. |
| **2** | 0,0 | - | 6,47 | 0,24 | 8 | 15 | 87 |
| MG-13-COx-062-P1 | 24,5 | 0,6 | 5,40 | 0,23 | 27 | 2331 | n.d. |
| KOH with 0,4 g/L L-Cysteine-HCl | 50,3 | 0,5 | 4,79 | 0,26 | 128 | 3197 | n.d. |
| | 73,3 | 0,3 | 5,76 | 0,22 | - | - | |
| | 92,3 | 0,3 | 6,32 | 0,22 | 150 | 5067 | n.d. |

### EXAMPLE 1b

The example 1 above was redone with only a difference in the shaking speed on the septum bottles for autotrophic productions. The shaking speed used was 100 min⁻¹.

A reporting error in example 1 was noticed where the results of cysteine for reactor 1 and 2 were switched. The actual results obtained in this example are provided in Table 1 b.

**Table 1 b. Results of acetate and ethanol production with different cysteine concentrations. (n.d. not detected)**

| | | | | | **NMR** | | |
|---|---|---|---|---|---|---|---|
| **Reactor** | **Time, h** | Δ**p, bar** | **pH** | **OD600** | **Ethanol, mg/L** | **Acetate, mg/L** | **Cysteine, mg/L** |
| **1** | 0,0 | - | 6,47 | 0,25 | 6 | 13 | 87 |
| MG-13-COx-062-P2 | 24,5 | 0,6 | 5,63 | 0,25 | 19 | 1418 | n.d. |
| KOH with 0,1 g/L L-Cysteine-HCl | 50,3 | 0,6 | 4,71 | 0,30 | 130 | 2556 | n.d. |
| | 73,3 | 0,6 | 4,83 | 0,30 | - | - | |
| | 92,3 | 0,4 | 5,90 | 0,26 | 321 | 6960 | n.d. |
| | | | | | | | |
| **2** | 0,0 | - | 6,47 | 0,24 | 8 | 15 | 199 |
| MG-13-COx-062-P1 | 24,5 | 0,6 | 5,40 | 0,23 | 27 | 2331 | 92 |
| KOH with 0,4 g/L L-Cysteine-HCl | 50,3 | 0,5 | 4,79 | 0,26 | 128 | 3197 | n.d. |
| | 73,3 | 0,3 | 5,76 | 0,22 | - | - | |
| | 92,3 | 0,3 | 6,32 | 0,22 | 150 | 5067 | n.d. |

### EXAMPLE 2

### Autotrophic production in carbonate buffer of C. ljungdahlii and synthesis gas with 0.4 g/L L-Cysteine.HCl and no L-Cysteine.HCl

The wild-type strain *Clostridium ljungdahlii* was used under growth-limiting conditions for the autotrophic product formation in carbonate buffer with 0.4 g/L of L-cysteine hydrochloride.

For the production, anaerobic carbonate buffer (adjusted with KOH 1.87 g / L) was used. The only addition to the buffer was L-cysteine-HCl at 0.4 g/L concentration or no addition at all.

Identical autotrophic productions were carried out in 500 mL septum bottles with 100 mL of carbonate carried out in a shaking water bath shaker (open Innova 3100 by New Brunswick Scientific) at 100 min⁻¹. The productions were carried out at 37 °C with manual control of the pH. Once the pH dropped below 5.5, KOH was metered from a stock solution of 187 g / L. In the present experiments the pH in both reactors at points in time of 50.3 h and 73.3 h were taken and corrected by the addition of each 1.87 g/L KOH manually.

The reactors were inoculated with a synthesis gas mixture of 67% H₂ and 33 % CO₂ pressurized with an overpressure of 0.8 bar. To replenish the consumed gas, the gas atmosphere was completely replaced once a day in the headspace of the reactors and again pressured to 0.8 bars.

The reactor 1 contained a concentration of L-cysteine HCl of 0 g/L, and reactor 2 a concentration of L-cysteine HCl of 0.4 g / L.

The reactors started with autotrophic cells at an OD of 0.2. The pre-culture was carried out continuously in a 1 L bottle with 500 ml of complex medium. The complex medium comprised of 1 g/l NH₄Cl, 0.1 g/l KCI, 0.2 g/l MgSO₄ x7 H₂O, 0.8 g/l NaCl, 0.1 g/l KH₂PO₄, 20 mg/l CaCl₂ x 2 H₂O, 20 g/l MES, 1 g/l yeast extract, 0.4 g/L L-cysteine HCl, 20 mg/l nitrilotriacetic acid, 10 mg/l MnSO₄ H₂O x, 8 mg/l (NH₄)₂Fe(SO₄)₂ x 6 H₂O, 2 mg/L CoCl₂ x 6 H₂O, 2 mg/l ZnSO₄ x 7 H₂O, 0.2 mg/l CuCl₂ × 2H₂O, 0.2 mg/l Na₂MoO₄ x 2 H₂O, 0.2 mg/l NiCl₂ x 6 H₂O, 0.2 mg/l Na₂SeO₄, 0.2 mg/l Na₂WO₄ x 2 H₂O, 20 µg/l d-biotin, 20 µg/L folic acid, 100 µg/l pyridoxine HCl, 50 µg/l thiamine-HCl x H₂O. 50 µg/l of riboflavin, 50 µg/l nicotinic acid, 50 µg/l Ca-pantothenate, 1 µg/L of vitamin B12, 50 µg/l p-aminobenzoate, 50 µg/l lipoic acid. The gassing of the culture with synthesis gas (67 % H₂, 33 % CO₂) was carried out continuously at a flow rate of 3 l/h over a filter with pore size of 10 microns. The cells were in late log phase of growth at an OD of 0.23 anaerobically centrifuged (4500 rpm, 4300 g, 20 °C, 10 min). The supernatant was discarded and the cell pellet resuspended in 10 ml carbonate buffer. The prepared cells were then used to inoculate the actual experiments.

When sampling each 5 ml sample was removed for determination of OD₆₀₀, pH and the product range. The determination of the product concentration was performed by quantitative ¹H-NMR spectroscopy. As an internal quantification standard sodium trimethylsilylpropionate (T(M)SP) was used.

In both reactors gas consumption was measured from the start, the gas consumption in reactor 2 with an L-cysteine-HCl concentration of 0.4 g/L over the test period of 98.8 h was higher than in the reactor with an L-cysteine-HCl concentration of 0 g/L. The concentrations of ethanol and acetate formed with an L-cysteine-HCl concentration of 0.4 g/ L at the end of the 98.8 h reaction time was higher than that produced in the reactor where no L-cysteine-HCl was added.

**Table 2. Product formation of cultures of Clostridium ljungdahlii in complex medium on synthesis gas with 67% H₂ and 33 % CO₂ with different initial concentrations of L-cysteine.**

| | | | | | **NMR** | | |
|---|---|---|---|---|---|---|---|
| **Reactor** | **Time, h** | **Δp, bar** | **pH** | **OD₆₀₀** | **Ethanol, mg/L** | **Acetate, mg/L** | **Cysteine, mg/L** |
| **1** | 0,0 | | 6,73 | 0,17 | 5 | 4 | n.d. |
| MG-13-COx-056-P3 | 26,0 | 0,3 | 6,45 | 0,15 | 5 | 380 | n.d. |
| KOH without L-Cysteine-HCl | 50,3 | 0,2 | 6,33 | 0,13 | 10 | 822 | n.d. |
| | 74,3 | 0 | 6,15 | 0,12 | | | |
| | 98,8 | 0 | 6,13 | 0,12 | 7 | 640 | n.d. |
| | | | | | | | |
| **2** | 0,0 | | 6,7 | 0,18 | 5 | 15 | 396 |
| MG-13-COx-056-P1 | 26,0 | 0,6 | 6,02 | 0,18 | 21 | 1714 | 35 |
| KOH with 0,4 g/L L-Cysteine-HCl | 50,3 | 0,5 | 5,13 | 0,19 | 91 | 2471 | n.d. |
| | 74,3 | 0,4 | 6,17 | 0,18 | | | |
| | 98,8 | 0,2 | 5,87 | 0,18 | 84 | 3366 | n.d. |

| | | | | | | | |
|---|---|---|---|---|---|---|---|
| (n.d. =not detected) | | | | | | | |

### EXAMPLE 3

### Formation of acetic acid and ethanol from synthesis gas with Clostridium ljungdahlii and refeeding of cysteine

For the biotransformation of hydrogen and carbon dioxide to acetic acid and ethanol the homoacetogenic bacterium *Clostridium Ijungdahlii* was cultivated on synthesis gas with refeeding of cysteine. All cultivation steps were carried out under anaerobic conditions in pressure-resistant glass bottles that can be closed airtight with a butyl rubber stopper.

For the preculture of *C. ljungdanlii* 500 ml medium (ATCC1754-medium: pH = 6.0; 20 g/L MES; 1 g/L yeast extract, 0.8 g/L NaCl; 1 g/L NH₄Cl; 0.1 g/L KCI; 0.1 g/L KH₂PO₄; 0.2 g/L MgSO₄ x 7 H₂O; 0.02 g/L CaCl₂ x 2 H₂O; 20 mg/L nitrilotriacetic acid; 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-biotin; 20 µg/L folic acid; 100 µg/L pyridoxine-HCl; 50 µg/L thiamine-HCl x H₂O; 50 µg/L riboflavin; 50 µg/L nicotinic acid; 50 µg/L Ca-pantothenate; 1 µg/L vitamin B₁₂; 50 µg/L p-aminobenzoate; 50 µg/L lipoic acid; approx. 67.5 mg/L NaOH) with additional 400 mg/L L-cysteine-hydrochloride and 400 mg/L Na₂S x 9 H₂O were inoculated with 5 mL of a frozen cryo stock. The chemolithoautotrophic cultivation was carried out in a 1 L pressure-resistant glass bottle at 37°C, 100 rpm and a ventilation rate of 3 L/h with a premixed gas with 67% H₂, 33% CO₂ in an open water bath shaker for 69 h till OD₆₀₀ₙₘ >0.4. The gas was discharged into the medium through a sparger with a pore size of 10 µm, which was mounted in the center of the reactors. Then the cell suspension was centrifuged and the cell pellet was resuspended in fresh CGF1 mineral medium as disclosed below.

For the production phase, as many washed cells from the preculture of *C. ljungdahlii* as necessary for an OD₆₀₀ₙₘ of 0.2 were added to 100 ml mineral medium (CGF1 medium, pH 6.5, 1.4 g/L KOH, 2 g/L (NH₄)₂SO₄, 1 g/L KH₂PO₄, 1 g/L K₂HPO₄, 10 mg/L FeSO₄ X 7 H₂O, 3.8 mg/L MnSO₄ X 1 H₂O, 246 mg/L MgSO₄ x 7 H₂O, aerated for 30 min with a premixed gas with 67% H₂ and 33% CO₂). At the beginning, culture A (s shown in table 3) was supplemented with additional 400 mg/L L-cysteine-hydrochloride and cultures B and C with 200 mg/L L-cysteine-hydrochloride each. The cultivation was carried out in 500 mL pressure-resistant glass bottles at 37°C, 150 rpm in an open water bath shaker for 163 h which were aerated to an overpressure of 0.8 bar with a premixed gas with 67% H₂, 33% CO₂ one time a day. The pH was held >5.0 by intermittent additions of a 140 g/L KOH solution. During the cultivation several 5 mL samples were taken to determinate OD₆₀₀ₙₘ, pH und product formation. Culture C was re-fed with 200 mg/l L-cysteine-hydrochloride after 18, 41, 65 and 89 h of cultivation. The determination of the product concentrations including the L-Cysteine concentration was performed by semiquantitative ¹H-NMR spectroscopy. As an internal quantification standard sodium trimethylsilylpropionate (T(M)SP) was used.

During the production phase the concentrations of acetate and ethanol in culture C increased more than in culture A and B (see table 3), also culture C had a stronger cell growth than cultures A and B. In all three cultures all the added L-cysteine was consumed completely.

**Table 3: Cell growth and product formation of cultures of Clostridium ljungdahlii in CGF1 mineral medium on synthesis gas with 67% H₂ and 33% CO₂ with different initial concentrations of L-cysteine and partial refeeding of L-cysteine.**

| **Culture** | **Time [h]** | **OD(600nm)** | **Acetate [mg/L]** | **Ethanol [mg/L]** | **L-Cysteine [mg/L]** | **Feed [mg/L Cys-HCl)** |
|---|---|---|---|---|---|---|
| | 0 | 0,206 | 34,0 | 2,0 | 270,0 | - |
| | 18,75 | 0,232 | 1400,0 | 60,0 | 70,0 | - |
| | 41,5 | 0,293 | 2550 | 420 | n.d. | - |
| **A** | 46,75 | 0,281 | 2700 | 480 | n.d. | - |
| | 65,5 | 0,324 | 3500 | 580 | n.d. | - |
| | 69,5 | 0,366 | 3700 | 650 | n.d. | - |
| | 89,75 | 0,324 | 4350,0 | 580,0 | n.d. | - |
| | 95,5 | - | - | - | n.d. | - |
| | 163,5 | 0,339 | 6100,0 | 670,0 | n.d. | - |
| **B** | 0 | 0,197 | 35,0 | 1,9 | 132,5 | - |
| | 18,75 | 0,261 | 1400,0 | 100,0 | n.d. | - |
| | 41,5 | 0,279 | 2150 | 580 | n.d. | - |
| | 46,75 | 0,269 | 2200 | 630 | n.d. | - |
| | 65,5 | 0,271 | 2200 | 600 | n.d. | - |
| | 69,5 | 0,259 | 2200 | 590 | n.d. | - |
| | 89,75 | 0,241 | 2350,0 | 560,0 | n.d. | - |
| | 95,5 | - | - | - | n.d. | - |
| | 163,5 | 0,237 | 2550,0 | 570,0 | n.d. | - |
| | 0 | 0,209 | 27,0 | 2,0 | 120,0 | - |
| | 18,75 | 0,245 | 1400,0 | 85,0 | n.d. | 200 |
| | 41,5 | 0,339 | 3000 | 510 | n.d. | 200 |
| **C** | 46,75 | 0,335 | 3250 | 580 | 123,0 | - |
| | 65,5 | 0,399 | 4250 | 790 | n.d. | 200 |
| | 69,5 | 0,392 | 4400 | 860 | 108,0 | - |
| | 89,75 | 0,431 | 5000,0 | 810,0 | n.d. | 200 |
| | 95,5 | 0,434 | 5600,0 | 880,0 | 89,0 | - |
| | 163,5 | 0,481 | 7700,0 | 960,0 | n.d. | - |

| | | | | | | |
|---|---|---|---|---|---|---|
| (n.d. =not detected) | | | | | | |

## Claims

1. A method of culturing at least one acetogenic bacteria, the method comprising growing the bacteria in an aqueous medium comprising an amount of cysteine and/or cystine, wherein the cysteine and/or cystine concentration in the medium is maintained at a concentration range of 0.05g/L to 0.20g/L.

2. The method according to claim 1, wherein the concentration of cysteine and/or cystine in the medium is maintained at about 0.10g/L.

3. The method according to either claim 1 or 2, wherein the concentration of cysteine and/or cystine in the medium is maintained at about 0.08g/L.

4. The method according to any one of the preceding claims, wherein the medium further comprises a carbon source comprising CO and/or CO₂.

5. The method according to claim 4, wherein the carbon source comprises at least 50% by weight of CO and/or CO₂.

6. The method according to any of the preceding claims, wherein the acetogenic bacteria is selected from the group consisting of wherein the first microorganism is selected from the group consisting of *Acetoanaerobium notera (ATCC 35199), Acetonema longum (DSM 6540), Acetobacterium carbinolicum (DSM 2925), Acetobacterium malicum (DSM 4132), Acetobacterium species no. 446, Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797, Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1, Moorella thermoacetica (DSM 521), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440), Thermoanaerobacter kivui (DSM 2030).*

7. A method of producing acetate and/or ethanol from at least one carbon source, the method comprising the step of growing at least one acetogenic bacteria in an aqueous medium comprising an amount of cysteine and/or cystine, wherein the concentration of cysteine and/or cystine in the medium is maintained at a concentration range of 0.05g/L to 0.20g/L.

8. The method according to claim 7, wherein the concentration of cysteine and/or cystine in the medium is maintained at about 0.10g/L.

9. The method according to claim 7, wherein the concentration of cysteine and/or cystine in the medium is maintained at about 0.08g/L.

10. The method according to any one of the claims 7 to 9, wherein the carbon source comprises at least 50% by weight of CO and/or CO₂.

11. The method according to any one of the claims 7 to 10, wherein the acetogenic bacteria is selected from the group consisting of wherein the first microorganism is selected from the group consisting of *Acetoanaerobium notera (ATCC 35199), Acetonema longum (DSM 6540), Acetobacterium carbinolicum (DSM 2925), Acetobacterium malicum (DSM 4132), Acetobacterium species no. 446, Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797, Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1, Moorella thermoacetica (DSM 521), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440), Thermoanaerobacter kivui (DSM 2030).*

12. The method according to any one of the claims 7 to 11, wherein the cysteine and/or cystine present in the medium is added as a compound selected from the group consisting of L-cysteine-HCl, L-Cysteine, L-Cysteine-HCl (free of water) and L-Cysteine*H₂O.

13. A method of fermenting syngas to produce acetate and/or ethanol, the method comprising culturing at least one acetogenic bacteria in an aqueous medium comprising an amount of cysteine and/or cystine, wherein the cysteine and/or cystine is maintained at a concentration range of 0.05g/L to 0.20g/L.

14. The method according to claim 13, wherein the concentration of cysteine and/or cystine in the medium is maintained at about 0.10g/L.

15. The method according to either one of the claims 13 or 14, wherein the acetogenic bacteria is selected from the group consisting of wherein the first microorganism is selected from the group consisting of *Acetoanaerobium notera (ATCC 35199), Acetonema longum (DSM 6540), Acetobacterium carbinolicum (DSM 2925), Acetobacterium malicum (DSM 4132), Acetobacterium species no. 446, Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797, Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1, Moorella thermoacetica (DSM 521), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440), Thermoanaerobacter kivui (DSM 2030).*
